# FASCICULE DE BREVET EUROPEEN

(11) **EP 3 326 475 B1**
(45) Date de publication et mention de la délivrance du brevet: **12.02.2020**
(21) Numéro de dépôt: 17200483.0
(22) Date de dépôt: 08.11.2017
(51) Int. Cl.: A41F 9/02, A41D 1/06

(54) **VÊTEMENT DESTINÉ À LA PRATIQUE DU SPORT**
KLEIDUNGSSTÜCK FÜR DAS PRAKTIZIEREN VON SPORT
SPORTS GARMENT

(30) Priorité: 23.11.2016 FR 1661384
(43) Date de publication de la demande: 30.05.2018
(73) Titulaire: Rossignol Apparel, 38430 Saint Jean de Moirans (FR)
(72) Inventeur: VAUGE-LALANNE, Magali, 73120 COURCHEVEL VILLAGE (FR); LASALLE, Florent, 38500 SAINT CASSIEN (FR); KRUAJITCH, André-Jean, 38500 LA BUISSE (FR)
(74) Mandataire: Cabinet Laurent & Charras

(56) Documents cités:
- US-A1- 2004 163 159
- US-A1- 2011 009 793
- US-A1- 2014 273 734
- US-A1- 2014 273 743
- US-A1- 2015 366 270
- US-A1- 2016 050 986
- US-A1- 2016 106 157

## Description

### Domaine technique

L'invention se rattache au domaine de l'habillement, et plus précisément à celui des vêtements utilisés dans le cadre de pratiques sportives, et plus spécifiquement de la pratique des sports en extérieur, dans des conditions atmosphériques qui peuvent être contraignantes. Elle trouve une application toute particulière pour le domaine des sports de glisse sur neige, de la randonnée ou de la course à pied.

Elle vise plus particulièrement une nouvelle conception de vêtements, qui incluent des zones de compression destinées en particulier à favoriser la mobilisation de la chaîne musculaire active lors de la pratique de certains sports.

### Techniques antérieures

De façon générale, les vêtements employés pour la pratique des sports de glisse sur neige sont conçus pour assurer un confort thermique à leur utilisateur, notamment en empêchant la pénétration d'air froid ou de neige dans les zones qu'ils recouvrent. Pour une pratique sportive plus intense, il est également reconnu qu'il est avantageux d'exercer une certaine compression ou au moins un maintien sur les muscles les plus actifs lors des mouvements ou des positions adoptés pendant une pratique spécifique. Cette compression accélère le temps de contraction des muscles, diminue les vibrations musculaires au cours de l'effort, et permet une meilleure oxygénation des muscles et a un effet favorable sur l'élimination des déchets produits lors de l'activité musculaire.

Des solutions ont d'ores et déjà été proposées pour assurer cet effet de compression dans différents types de vêtements. Ainsi, certains pantalons ou sous-vêtements destinés à recouvrir le bas du corps sont réalisés en matériau élastique, et sont conçus pour que ce matériau soit tendu lorsque l'utilisateur porte le vêtement. Le document US 2016/0106157 décrit un exemple de cette solution, dans laquelle le vêtement comporte une fraction élastique, assurant une compression de la partie du corps qu'il recouvre, et une couche extérieure relativement ample, dissimulant la fraction élastique. On conçoit toutefois que l'application permanente de cette compression peut être gênante pour l'utilisateur principalement lors des phases de repos ou d'attente, et qu'il soit nécessaire de pouvoir ne l'activer que lorsque l'utilisateur le souhaite.

Pour ce faire, on a proposé par exemple dans le document EP 1 802 208 d'équiper le vêtement de zones qui sont coulissantes à l'intérieur de fourreaux formés sur le vêtement. Un déplacement de l'extrémité de ces zones coulissantes permet de serrer de manière plus ou moins intense la zone recouverte par la pièce en question. Le blocage de cette extrémité se fait au moyen de bandes de type Velcro®. La manipulation de ce type de système de verrouillage n'est pas particulièrement aisée, en particulier pour un utilisateur équipé de gants. Par ailleurs, les zones coulissantes ne présentent pas forcément une élasticité qui leur permet de se déformer lorsque le muscle se contracte, ce qui peut s'avérer inconfortable pour l'utilisateur.

Une solution alternative a été décrite dans le document US 8 597 222. Le vêtement décrit dans ce document présente deux pans séparés, recouvrant respectivement l'avant et l'arrière de la cuisse, et solidarisés entre eux par des moyens réglables en position. Ces moyens peuvent être par exemple une bande de type Velcro® permettant de bloquer les deux surfaces de textile venant en regard lorsque les deux pans textiles sont tendus pour recouvrir la cuisse. On conçoit que la compression ainsi obtenue n'est pas facilement réglable en cours d'utilisation du vêtement, et très peu confortable lorsque les conditions atmosphériques sont défavorables. D'autre part, le réglage n'est pas toujours reproductible d'une utilisation à l'autre.

D'autres solutions de réglage sont prévues dans ce document, par des systèmes d'enroulement d'une bande textile, ou encore un mécanisme de laçage visant à permettre le rapprochement des deux pans indépendants de textile. Ces systèmes ne sont pas réellement satisfaisants car ils forment des surépaisseurs importantes du vêtement qui, outre le côté inesthétique, peuvent être inconfortables. Le document US 2014/273734 divulgue une robe dans laquelle une seule fermeture (90) permet de fermer l'arrière de la robe pour mettre en tension à la fois la doublure interne et la couche externe de la robe.

### Exposé de l'invention

Un problème que se propose de résoudre l'invention est celui du réglage de la compression exercée par un vêtement sur le corps de l'utilisateur, qui soit facilement accessible lorsque les conditions atmosphériques sont défavorables, y compris lorsque l'utilisateur a une précision de dextérité limitée, par exemple parce qu'il porte des gants. Un objectif de l'invention est de permettre ce réglage sans générer d'inconfort pour l'utilisateur, et de préférence sans dégrader l'esthétique du vêtement.

Pour ce faire, le Demandeur a conçu un vêtement destiné à la pratique du sport qui comporte une couche extérieure et une doublure interne. Cette doublure interne inclut une région élastique, permettant d'exercer un effort de compression sur la zone qu'elle recouvre.

Conformément à l'invention ce vêtement se caractérise en ce que la doublure interne comporte une fermeture à glissière située sous la couche extérieure, dont au moins la première bande est solidarisée à cette zone élastique, cette zone élastique étant maintenue plaquée contre la zone du corps lorsque la fermeture à glissière est fermée.

En d'autres termes, l'invention consiste à équiper le vêtement d'une zone élastique dont la tension est déterminée par la position d'une fermeture à glissière reliant cette zone élastique au reste du vêtement. Autrement dit, lorsque la fermeture à glissière est ouverte, la zone élastique est détendue, et aucune compression supplémentaire n'est exercée par le vêtement sur le corps de l'utilisateur, qui reste toutefois protégé par la couche extérieure. A l'inverse, lorsque la fermeture à glissière est fermée, la zone élastique est tendue, et la compression recherchée est exercée sur le corps de l'utilisateur.

En pratique, dans une première forme de réalisation, la seconde bande de la fermeture à glissière peut être solidaire de la zone élastique. Ainsi, la fermeture de la glissière rapproche deux pans de la zone élastique, et assure ainsi sa mise sous tension. Autrement dit, la fermeture caractéristique est localisée intégralement sur la doublure interne et permet la modification de la géométrie de cette dernière lorsqu'elle est actionnée.

Dans une autre forme de réalisation, la seconde bande de la fermeture à glissière peut être solidaire de la couche extérieure. En d'autres termes, la fermeture de la glissière rapproche un des pans de la zone élastique de la couche extérieure du vêtement qui constitue une région fixe par rapport à laquelle la zone élastique est tendue. Autrement dit, la fermeture caractéristique assure la liaison entre la doublure interne élastique et la couche extérieure.

Avantageusement en pratique, le vêtement peut comporter une pièce textile reliant les bandes de la fermeture à glissière. Cette pièce a des dimensions telles qu'elle n'est pas tendue lorsque la fermeture à glissière est ouverte. Elle assure la continuité soit entre les deux pans de la bande textile, soit entre la bande textile et la couche extérieure, et agit ainsi en tant que soufflet.

Dans une forme avantageuse de réalisation, la fermeture à glissière peut présenter un curseur à blocage de position. En d'autres termes, le curseur est conçu pour rester dans une position intermédiaire quand cela est souhaité, et assurer ainsi une compression d'intensité intermédiaire entre la compression nulle lorsque la glissière est totalement ouverte, et la compression maximale lorsque la glissière est totalement fermée.

En fonction du type de vêtement, et pour faciliter l'ergonomie, le curseur peut se déplacer vers le haut ou vers le bas pour sa fermeture.

Avantageusement en pratique, le vêtement peut comporter une ouverture formée dans la couche extérieure, formant l'entrée d'un volume donnant accès à la fermeture à glissière associé à la région élastique. En d'autres termes, le vêtement comporte une sorte de poche à l'intérieur de laquelle se trouve le curseur de la fermeture à glissière caractéristique, de sorte que l'accès à cette dernière se fait après ouverture de cette poche, accessible depuis la face extérieure du vêtement.

Ceci permet l'activation de la zone élastique le moment voulu, tout en conservant une protection aux conditions atmosphériques assurées par les propriétés de la couche extérieure.

Avantageusement, l'ouverture formée dans la couche extérieure présente une fermeture à glissière qui est décalée par rapport à la fermeture à glissière solidaire de la zone élastique. En d'autres termes, l'entrée de la poche donnant accès à la fermeture à glissière caractéristique peut se faire par l'actionnement d'une autre fermeture à glissière qui est avantageusement positionnée pour éviter qu'elle ne se superpose à la fermeture à glissière agissant sur la zone élastique, et ainsi éviter le risque de surépaisseur qui peut être inconfortable.

L'invention trouve une application toute particulière pour la fabrication de vestes, et plus généralement de vêtements destinés à couvrir la partie haute du corps, pour assurer une compression de la région dorsale/lombaire et/ou abdominale. Dans ce cas, la région élastique est avantageusement disposée dans la zone destinée à recouvrir la taille de l'utilisateur.

Dans une forme particulière de réalisation, la veste peut ainsi comporter deux fermetures à glissière, disposées de chaque côté de la zone élastique, à proximité de l'ouverture principale du vêtement. Dans ce cas, la mise en tension de la zone élastique peut se faire lorsque le vêtement est encore ouvert, avec son ouverture centrale donnant accès aux fermetures à glissière caractéristiques, qui peuvent être fermées ou ouvertes avant que l'utilisateur ne referme le vêtement.

Avantageusement, la lisière haute et/ou la lisière basse de la zone élastique peuvent être cousues à la doublure interne du vêtement, de sorte que la ou les fermetures à glissière caractéristiques sont disposées sur les lisières latérales et verticales de la zone élastique.

L'invention peut également être appliquée à des vêtements de type pantalon, ou plus généralement des vêtements destinés à couvrir les membres inférieurs. Avantageusement, la région élastique peut être agencée pour entourer la cuisse de l'utilisateur.

### Description sommaire des figures

La manière de réaliser invention, ainsi que les avantages qui en découlent, ressortiront bien de la description des modes de réalisation qui suit, avec à l'appui des figures annexées dans lesquelles :
- la figure 1 est une vue de face d'une veste réalisée conformément à l'invention, dans laquelle la zone élastique est détendue ;
- la figure 2 est une vue de face de la veste de la figure 1, dans laquelle la zone élastique et partiellement tendue ;
- la figure 3 est une vue en coupe schématique selon le plan III-III' de la figure 2 ;
- la figure 4 est une vue de face d'un pantalon conforme invention, montré dans une configuration où le volume d'accès à la zone élastique caractéristique est ouvert ;
- la figure 5 est une vue de face du pantalon de la figure 4 montrant les zones élastique caractéristiques tendues ;
- la figure 6 est une vue analogue à la figure 4, dans laquelle une des zones élastique est partiellement tendue.
- la figure 7 est une vue en coupe selon le plan en VII-VII' de la figure 6

Bien entendu, ces dessins sont donnés uniquement à titre illustratif, et n'ont pour but que de faire comprendre les aspects essentiels de l'invention, de sorte que les vêtements représentés peuvent paraître très simplifiés par rapport à la réalité.

### Manières de réaliser l'invention.

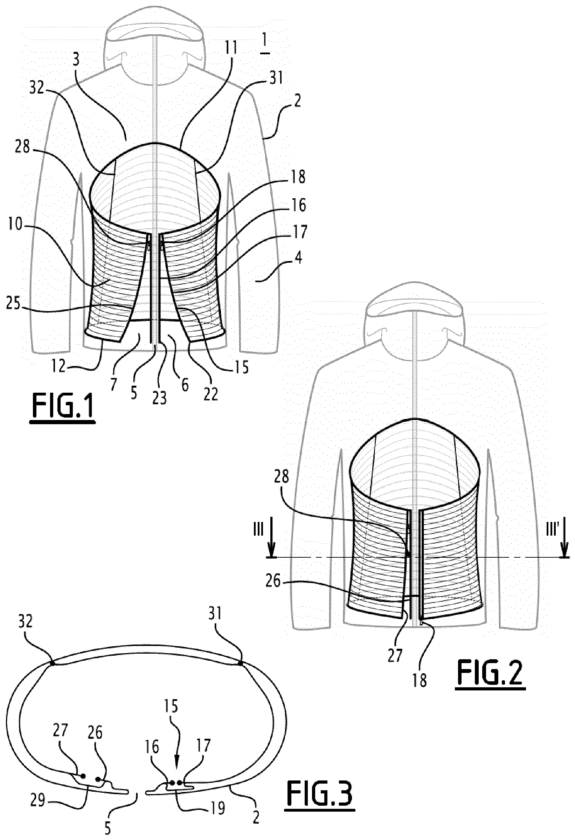

Comme déjà évoqué, l'invention peut trouver différentes applications, en particulier pour la fabrication d'une veste est illustrée à la figure 1.

Cette veste **1** comporte une couche extérieure **2** qui possède principalement une partie 3 recouvrant le torse et le dos, et des manches **4.** La partie **3** recouvrant le torse et le ventre présente dans la forme illustrée une ouverture centrale **5**, au niveau de laquelle les deux pans **6**, **7** se rejoignent, et peuvent être solidarisés par des moyens non représentés tels qu'une fermeture à glissière, des boutons, ou des boutons pression.

Conformément à l'invention, la veste **1** comprend une zone élastique **10**, destinée à venir assurer un effet de compression au niveau de l'abdomen, de la taille et de la zone dorsale dorso-lombaire. Pour ce faire, cette zone élastique **10** est composée d'un textile à base de fils élastiques, préférentiellement tissés. Ce tissu **10** et disposé de telle sorte qu'il présente une capacité d'allongement en direction horizontale, de telle sorte que la zone élastique **10** forme une ceinture élargie, qui entoure la partie ventrale de l'utilisateur. À titre d'exemple, le textile peut être composé d'élasthanne associé à du polyester ou du polyamide, l'élasthanne permettant de donner les propriétés d'élasticité au textile. Le pourcentage d'élasthanne utilisé est généralement supérieur à 15%, voire de préférence de l'ordre de 20%, en chaine (qui se trouve orientée horizontalement lorsque le vêtement est porté) et éventuellement également en trame. La compression appliquée sur le corps de l'utilisateur peut être comprise entre 4 et 6 mm Hg pour assurer le maintien des muscles. Elle peut éventuellement être plus importante, en particulier supérieure à 15 mm Hg pour exercer de la compression sur les muscles.

Conformément à l'invention, cette région élastique **10** est équipée de fermetures à glissière qui permettent d'assurer la mise en tension de la zone élastique **10**. Plus précisément, dans la forme illustrée, la zone élastique **10** comporte deux fermetures à glissière **15,25** orientées sensiblement verticalement (lorsque le vêtement est porté par une personne debout). Une première fermeture à glissière **15** est située sur un premier côté vertical de la zone élastique. Cette fermeture à glissière **15** comporte deux bandes **16,17** qui coopèrent l'une avec l'autre sous l'action du curseur **18**. Une première bande **16** est cousue ou plus généralement solidarisée à la couche extérieure, et dans la forme illustrée à proximité de l'ouverture centrale **5** de la veste. Cette bande **16** forme le point fixe par rapport à la couche extérieure de la veste. L'autre bande **17** est quant à elle cousue sur le bord de la bande élastique **10**. Une configuration symétrique se retrouve de l'autre côté de la veste, avec une fermeture à glissière **25** dont une première bande **26** est cousue sur la couche extérieure, tandis que l'autre bande **27** est cousue sur un côté vertical de la zone élastique **10**.

Dans la forme illustrée, la lisière haute **11** de la zone élastique **10** est solidarisée, par exemple cousue, sur la couche extérieure **2**, tandis que la lisière basse **12** est libre de manière à permettre la déformation plus aisée de la partie basse de la zone élastique **10**. Dans la forme illustrée aux figures 1 et 2, la zone élastique présente deux coutures sensiblement verticales **31**, **32** situées sur le panneau arrière de la doublure de la veste pour s'adapter au mieux à la forme du dos de l'utilisateur.

Dans la forme illustrée figure 1 et 2, les curseurs **18**, **28** des fermetures à glissière **15**, **25** se situent en position haute lorsque la fermeture à glissière est ouverte. Dans cette position ouverte, aucune pression n'est exercée sur les zones dorsale et ventrale de l'utilisateur et le coin inférieur **22** de la zone élastique s'écarte d'au moins 10cm, et de préférence d'une valeur comprise entre 15 et 20cm de l'extrémité basse **23** de la bande **16** de la fermeture à glissière **15**, lorsque le vêtement est porté. Comme l'illustre la figure 2, lorsque le curseur **18** est descendu en position basse, et que les deux bandes **26,27** sont assemblées, une tension est exercée sur la zone correspondante de la région élastique **10**.

Parallèlement, la fermeture à glissière **15** située de l'autre côté est partiellement fermée, avec un curseur **28** en position intermédiaire, de sorte que la région du corps en correspondance de la zone élastique **10** est moins contrainte.

Comme illustrée à la figure 3, les deux bandes **16**,**17** et **26**,**27** des fermetures à glissière sont reliées par un textile supplémentaire **19**,**29** soit très extensible, soit inélastique en tissu de type filet par exemple, qui permet d'éviter les mouvements inopinés de la lisière de la zone textile élastique lorsqu'elle est libre. Ce textile supplémentaire ne doit pas exercer de contraintes sur le vêtement ni en position ouverte, ni en position fermée. Aussi, la largeur de ce textile supplémentaire doit être supérieure à l'écartement entre les bandes obtenu après ouverture totale de la glissière, pour ne pas gêner à la libération de la compression sur le corps de l'utilisateur.

L'emploi de fermeture à glissière dont le curseur peut se bloquer en position intermédiaire permet de régler à volonté l'intensité de la compression appliquée sur le corps de l'utilisateur.

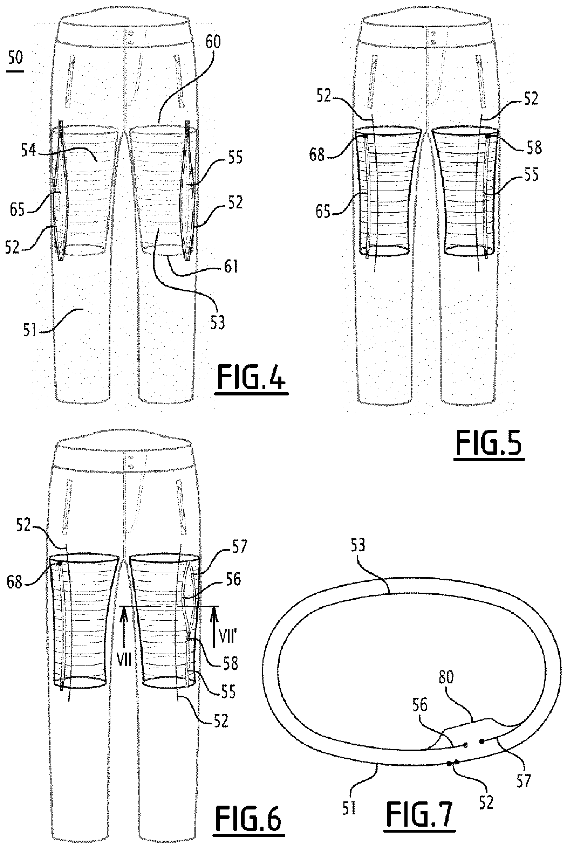

L'invention peut également trouver un intérêt particulier pour la fabrication de pantalons **50**, tel qu'illustré aux figures 4 à 7. Dans ce cas, la couche extérieure **51** présente des ouvertures **52** qui peuvent se fermer grâce à des fermetures à glissière comme illustré, ou bien encore deux bandes Velcro® analogues, pour donner accès aux fermetures à glissière **55**,**65** des zones élastiques **53**,**54** entourant la cuisse.

Plus précisément, ces zones élastiques **53**, **54**, peuvent être réalisées dans un tissu analogue à celui employé pour les zones élastiques de la veste des figures 1 à 3. La lisière haute **60** de ces bandes élastiques est cousue sur la couche extérieure **51** du pantalon, de manière à être maintenue en position au-dessus de la zone sur laquelle doit être appliquée la compression. La lisière basse **61** est également cousue à la couche extérieure **51** du pantalon, et la fermeture à glissière **55**,**65** s'ouvre en faisant coulisser le curseur **58,68** depuis le haut vers le bas.

Ainsi, lorsque les zones élastiques **53** ,**54** sont dans la position illustrée à la figure 5, sur laquelle les fermetures à glissière **55,65** sont fermées, elles exercent une compression maximale sur la cuisse de l'utilisateur. Lorsque l'utilisateur souhaite relâcher cette pression, il lui suffit d'ouvrir les fermetures à glissière **52** de la couche extérieure **51**, comme illustrée à la figure 4, afin d'accéder aux fermetures à glissière **55,65** associées aux zones élastiques **52,53,** comme l'illustre la figure 6, et de déplacer le curseur **58,68** comme il le souhaite pour régler la compression exercée. En position ouverte maximale, l'écartement maximal entre les deux parties des glissières **55,65** est compris entre 3 et 6cm, de préférence entre 4 et 5cm, pour obtenir un relâchement suffisant au repos.

De la même manière que pour la veste illustrée à la figure 3, la fermeture à glissière **55** est équipée d'un textile supplémentaire **80** formant un soufflet, évitant que les bandes **56,57** de la fermeture à glissière **55** ne s'écartent trop lorsque la tension est relâchée, alors que la fermeture à glissière **55** est ouverte. De préférence ce textile supplémentaire **80** est positionné du côté interne du vêtement pour éviter le contact direct de la glissière avec la jambe de l'utilisateur. Avantageusement en pratique, la fermeture à glissière **55** associée à la bande élastique est décalée de la surépaisseur formée par l'ouverture ménagée dans la couche extérieure, qui est réalisé par une fermeture glissière **52** dans la forme illustrée. Dans le mode de réalisation présenté, les fermetures à glissière intérieure **55** associée à la bande élastique et extérieure **52** liée à la couche extérieure sont sensiblement parallèles et sont décalées transversalement d'un décalage compris entre 10 et 30mm, de préférence d'environ 20mm, ce qui permet un accès facile à la glissière intérieure liée aux zones élastiques.

Bien entendu, le principe de l'invention peut être décliné sur d'autres vêtements, ou d'autres parties de vêtements. Elle peut par exemple être appliquée à un pantalon court ne couvrant que la cuisse, de type short, ou bien encore au niveau des manches d'une veste pour permettre la compression du bras, ou encore au niveau de la partie basse des jambes d'un pantalon, pour permettre la compression du bas de la jambe ou du mollet, ceci en fonction du sport ou de l'activité pratiquée.

De façon générale, les glissières intérieures liées aux zones élastiques sont de préférence orientées dans la direction verticale du vêtement pour permettre un réglage de la compression de la zone élastique sur les muscles dans la direction transversale du vêtement. Les glissières extérieures associées, liées à la couche extérieure du vêtement présentent de préférence une orientation également verticale mais d'autres orientations pourraient être retenues du moment que l'accès à la glissière intérieure est possible.

Il ressort de ce qui précède que les vêtements conformes à l'invention présentent l'avantage d'une part de faciliter le réglage de la compression appliquée sur les zones spécifiques du corps qu'elle recouvre, avec une facilité de manœuvre et d'autre part une reproductibilité du réglage de la compression d'une utilisation à l'autre. D'autre part, l'invention permet des possibilités de réglage approprié entre une position fermée appliquant un maintien des muscles optimal et une position ouverte où le maintien n'existe plus pour permettre le relâchement des muscles au repos ou même dans des positions intermédiaires de réglage du maintien des muscles pour que le vêtement s'adapte parfaitement aux besoin de l'utilisateur.

## Revendications

1. Vêtement (1,50) destiné à la pratique du sport, comportant une couche extérieure (2,51) et une doublure interne liée à la couche extérieure, ladite doublure incluant une région élastique (10,53,54), la doublure interne comportant une fermeture à glissière intérieure (15,25,55,65) entièrement située sous la couche extérieure, dont au moins la première bande (17,56) est solidarisée à la zone élastique (10 ,53,54), ladite zone élastique (10,53,54) étant maintenue plaquée contre une zone du corps de l'utilisateur lorsque la fermeture à glissière intérieure est fermée, **caractérisé en ce que** ladite fermeture à glissière intérieure (15,25,55,65) de ladite doublure interne est accessible depuis une ouverture (5, 52) formée dans la couche extérieure.

2. Vêtement selon la revendication 1, **caractérisé en ce que** ladite ouverture (5, 52) formée dans la couche extérieure est munie d'une fermeture à glissière extérieure permettant d'accéder à la fermeture à glissière intérieure (15,25,55,65) de la doublure interne.

3. Vêtement selon la revendication 1, **caractérisé en ce que** la seconde bande (57) de la fermeture à glissière intérieure (55) est solidaire de la zone élastique.

4. Vêtement selon la revendication 1, **caractérisé en ce que** la seconde bande (16) de la fermeture à glissière intérieure (15) est solidaire de la couche extérieure

5. Vêtement selon la revendication 1, **caractérisé en ce qu'**il comporte une pièce textile (19,29,79) reliant les bandes de la fermeture à glissière intérieure.

6. Vêtement selon la revendication 1, **caractérisé en ce que** la fermeture à glissière intérieure présente un curseur (58,68) se déplaçant vers le haut pour sa fermeture.

7. Vêtement selon la revendication 1, **caractérisé en ce que** la fermeture à glissière intérieure présente un curseur (18,28) se déplaçant vers le bas pour sa fermeture.

8. Vêtement selon la revendication 1, **caractérisé en ce que** la fermeture à glissière intérieure présente un curseur à blocage de positions.

9. Vêtement selon la revendication 1, **caractérisé en ce que** l'ouverture (5, 52) formée dans la couche extérieure (51) présente une fermeture à glissière qui est décalée par rapport à la fermeture à glissière intérieure (55,65) solidaire de la zone élastique (53,54).

10. Vêtement (1) selon l'une des revendications 1 à 9, du type veste, **caractérisé en ce que** la région élastique est disposée dans la zone destinée à recouvrir la taille de l'utilisateur.

11. Vêtement selon la revendication 10, **caractérisé en ce qu'**il comporte deux fermetures à glissière intérieure (15,25), disposées de chaque côté de la zone élastique, à proximité de l'ouverture principale (5) du vêtement.

12. Vêtement selon la revendication 10, **caractérisé en ce que** la lisière haute (11,60) et/ou la lisière basse (61) de la zone élastique est/sont cousues sur la couche extérieure.

13. Vêtement (50) selon l'une des revendications 1 à 9, du type pantalon, **caractérisé en ce que** la région élastique entoure la cuisse de l'utilisateur.

## Patentansprüche

1. Kleidungsstück (1, 50) für das Praktizieren von Sport, mit einer Außenschicht (2, 51) und einem mit dieser Außenschicht verbundenen Innenfutter, wobei dieses Futter einen elastischen Abschnitt (10, 53, 54) enthält, wobei das Innenfutter einen Innenreissverschluss (15, 25, 55, 65) enthält, der vollständig unter der Außenschicht liegt, wobei mindestens einer seiner Seitenstreifen (17, 56) fest mit dem elastischen Abschnitt (10 , 53, 54) verbunden ist, wobei dieser elastische Abschnitt (10, 53, 54) gegen einen Körperabschnitt des Trägers gedrückt wird, wenn der Innenreissverschluss geschlossen wird, **dadurch gekennzeichnet, dass** dieser Innenreissverschluss (15, 25, 55, 65) dieses Innenfutters über eine Öffnung (5, 52) erreichbar ist, die in der Außenschicht vorgesehen ist.

2. Kleidungsstück nach Anspruch 1, **dadurch gekennzeichnet, dass** die genannte, in der Außenschicht vorgesehene Öffnung (5, 52) mit einem Außenreissverschluss versehen ist, durch die der Innenreissverschluss (15, 25, 55, 65) des Innenfutters erreicht werden kann.

3. Kleidungsstück nach Anspruch 1, **dadurch gekennzeichnet, dass** der zweite Seitenstreifen (57) des Innenreissverschlusses (55) mit dem elastischen Abschnitt fest verbunden ist.

4. Kleidungsstück nach Anspruch 1, **dadurch gekennzeichnet, dass** der zweite Seitenstreifen (16) des Innenreissverschlusses (15) mit der Außenschicht fest verbunden ist.

5. Kleidungsstück nach Anspruch 1, **dadurch gekennzeichnet, dass** es einen textilen Abschnitt (19, 29, 79) enthält, der die Seitenstreifen des Innenreissverschlusses verbindet.

6. Kleidungsstück nach Anspruch 1, **dadurch gekennzeichnet, dass** der Innenreissverschluss über einen Schieber (58, 68) verfügt, der zum Verschließen nach oben gezogen wird.

7. Kleidungsstück nach Anspruch 1, **dadurch gekennzeichnet, dass** der Innenreissverschluss über einen Schieber (18, 28) verfügt, der zum Verschließen nach unten gezogen wird.

8. Kleidungsstück nach Anspruch 1, **dadurch gekennzeichnet, dass** der Innenreissverschluss über einen Schieber mit Positionssperre verfügt.

9. Kleidungsstück nach Anspruch 1, **dadurch gekennzeichnet, dass** die in der Außenschicht (51) vorgesehene Öffnung (5, 52) einen Reissverschluss aufweist, der gegenüber dem mit dem elastischen Abschnitt (53, 54) fest verbundenen Innenreissverschluss (55, 65) versetzt ist.

10. Kleidungsstück (1) nach einem der Ansprüche 1 bis 9, vom Typ Jacke, **dadurch gekennzeichnet, dass** der elastische Abschnitt sich in der Zone befindet, die die Taille des Trägers bedeckt.

11. Kleidungsstück nach Anspruch 10, **dadurch gekennzeichnet, dass** es zwei Innenreissverschlüsse (15, 25) aufweist, die beiderseits des elastischen Abschnitts in der Nähe der Hauptöffnung (5) des Kleidungsstücks angeordnet sind.

12. Kleidungsstück nach Anspruch 10, **dadurch gekennzeichnet, dass** die obere Kante (11, 60) und/ oder die untere Kante (61) des elastischen Abschnitts auf die Außenschicht genäht ist/ sind.

13. Kleidungsstück (50) nach einem der Ansprüche 1 bis 9, vom Typ Hose, **dadurch gekennzeichnet, dass** der elastische Abschnitt den Schenkel des Trägers umgibt.

## Claims

1. Garment (1,50) intended for the practice of sport, comprising an outer layer (2,51) and an inner lining bonded to the outer layer, the said lining comprising an elastic region (10,53,54), with the inner lining comprising an inside zipper (15,25,55,65) located entirely under the outer layer, wherein at least the first strip (17,56) is made integral with the elastic zone (10 ,53,54), the said elastic zone (10,53,54) being held against an area of the user's body when the lower zipper is closed, **characterized in that** the said inside zipper (15,25,55,65) of the said inner lining can be reached through a lower opening (5,52) made in the outer layer.

2. Garment according to claim 1, **characterized in that** said opening (5, 52) formed in the outer layer is provided with an outside zipper allowing access to the inside zipper (15, 25, 55, 65) of the inner lining.

3. Garment according to claim 1, **characterized in that** the second strip (57) of the inside zipper (55) is integral with the elastic zone.

4. Garment according to claim 1, **characterized in that** the second strip (16) of the inside zipper (15) is integral with the outer layer

5. Garment according to claim 1, **characterized in that** it comprises a textile part (19,29,79) connecting the strips of the inside zipper.

6. Garment according to claim 1, **characterized in that** the inside zipper has a slider (58,68) moving upwards to close it.

7. Garment according to claim 1, **characterized in that** the inside zipper has a slider (18,28) moving downwards to close it.

8. Garment according to claim 1, **characterized in that** the inside zipper has a position locking slider.

9. Garment according to claim 1, **characterized in that** the opening (5, 52) made in the outer layer (51) is provided with an outside zipper which is offset with respect to the inside zipper (55, 65) integral with the elastic zone (53, 54).

10. Garment (1) according to one of claims 1 to 9, of the jacket type, **characterized in that** the elastic region is located in the area intended to cover the waist of the user.

11. Garment according to claim 10, **characterized in that** it has two inside zippers (15,25), arranged on each side of the elastic zone, near the main opening (5) of the garment.

12. Garment according to claim 10, **characterized in that** the upper hem (11,60) and/or lower hem (61) of the elastic zone is/are sewn to the outer layer.

13. Garment (50) according to one of claims 1 to 9, of the trousers type, **characterized in that** the elastic region encloses the thigh of the user.
